Europäisches Patentamt

European Patent Office

Office européen des brevets

(19)

(11) Numéro de publication : **0 278 816 B1**

(12) **FASCICULE DE BREVET EUROPEEN**

(45) Date de publication du fascicule du brevet :
16.10.91 Bulletin 91/42

(21) Numéro de dépôt : **88400115.7**

(22) Date de dépôt : **20.01.88**

(51) Int. Cl.⁵ : **A61F 5/44**, B65D 33/16

(54) Clamp perfectionné pour fermeture de poche de recueil de déchets corporels.

(30) Priorité : 30.01.87 FR 8701119

(43) Date de publication de la demande :
17.08.88 Bulletin 88/33

(45) Mention de la délivrance du brevet :
16.10.91 Bulletin 91/42

(84) Etats contractants désignés :
DE GB IT

(56) Documents cités :
GB-A- 2 076 881
US-A- 4 296 529
US-A- 4 551 888

(73) Titulaire : **LABORATOIRES BIOTROL**
**1, rue du Foin**
**F-75140 Paris Cedex 03 (FR)**

(72) Inventeur : **Holtermann, Henri**
**12, Avenue Pierre Loti**
**F-64500 Saint Jean de Luz (FR)**
Inventeur : **Hamelin, Claude**
**1 Lotissement Aquerrondo Chemin de Ferres**
**F-64310 Ascain (FR)**

(74) Mandataire : **Brycman, Jean et al**
**CABINET ORES 6, Avenue de Messine**
**F-75008 Paris (FR)**

# Description

L'invention a pour objet un clamp pour fermeture de poche de recueil de déchets corporels du type de celles utilisées par des individus ayant subi des opérations chirurgicales comme des iléostomies ou analogues.

On sait que ces poches de recueil, formées par un sachet d'un film ou feuille de matière plastique et munies sur une de leurs faces d'une ouverture propre à être reliée à l'ouverture d'ostomie de la paroi abdominale d'une patient, sont soit du genre à "jeter" soit du genre à "vidange". Pour ces dernières, qui sont utilisées en particulier lors des soins post-opératoires, ou par des personnes ayant subi une iléostomie, on prévoit d'associer à chaque poche un clamp amovible de serrage à étanchéité de la ou des feuilles constitutives de la poche, à une extrémité de celle-ci distante de celle de collecte des déchets corporels procurant ainsi une fermeture qui peut être retirée par une infirmière ou le patient lui-même pour vidanger la poche sans qu'il soit nécessaire de la désolidariser de la paroi abdominale où elle est fixée ou maintenue.

On connaît déjà de nombreuses réalisations, de tels clamps, par exemple par GB-A-2076881 ou par US-A-3,523,534. Le dispositif selon ce dernier document est du type à "guillotine", c'est-à-dire est constitué par deux parois parallèles d'un corps entre lesquelles peut pénétrer, par pivotement autour d'une charnière, une lame autour de laquelle sont repliés les bords de l'ouverture de la poche de recueil, les dimensions de la lame, du corps et des feuilles étant choisies de manière telle que la poche est fermée à étanchéité par pincement avec serrage de ses bords lorsque la lame est entièrement logée à l'intérieur desdites parois. Un accrochage à déclic de la lame sur le corps, à l'extrémité opposée à celle de pivotement, interdit toute ouverture intempestive. Un tel clamp est d'actionnement parfois malaisé, en particulier à l'ouverture, étant donné que pour permettre l'évacuation des déchets ou excréments hors de la poche il faut déplier l'extrémité de celle-ci, après décrochage de la lame ce qui peut être une source de gêne importante.

En outre, un tel dispositif n'est que peu ou pas déformable dans sa condition de fermeture en laquelle il présente trois parties parallèles, à savoir les deux parois et la lame logée entre celles-ci.

On connaît également une réalisation d'un type différent, dans laquelle le clamp est constitué par moulage de matière plastique suivant deux branches articulées entre elles autour d'une charnière-film avec, pour le maintien des branches accolées l'une contre l'autre en condition de fermeture, un moyen d'assemblage à déclic prévu aux extrémités opposées à celles d'articulation. Un tel dispositif décrit par exemple dans US-A-4,551,888, prévoit de serrer les bords libres de la poche de recueil entre de multiples nervures des deux branches dont la structure, comme celle des moyens d'assemblage, est ainsi relativement complexe.

On connaît enfin, par US-A-4.296.529, un clamp selon le préambule de la Revendication. Dans un tel clamp, où l'on ne prévoit pas d'effet de verrouillage propre aux branches articulées, on confère auxdites branches une certaine rigidité qui s'oppose, bien entendu, à leur déformation.

Le problème se pose donc de fournir un clamp perfectionné qui soit simultanément d'une grande facilité d'utilisation, totalement sûr en ce qui concerne la fermeture qu'il procure de la poche de recueil de déchets corporels qu'il est destiné à équiper et qui, en outre, puisse être au moins légèrement déformable sans inconvénients dans sa condition de fermeture, autorisant une certaine adaptation à la morphologie de son utilisateur.

Ce problème est résolu, dans un dispositif moulé d'une pièce en matière plastique et comportant deux branches articulées entre elles autour d'une charnière-film, lesdites branches étant conformées pour assurer, en condition opératoire, le serrage entre elles des films ou feuilles constitutifs de la poche de recueil sur le bord libre de vidange de celle-ci, avec des moyens d'immobilisation amovible des branches l'une par rapport à l'autre, lesdites branches à contour plan sensiblement rectangulaire étant conformées suivant les parties mâle et femelle d'un accouplement à engagement, d'une part, et ménageant à leur extrémité opposée à celle d'articulation, un moyen de sécurité à engagement à pincement élastique, d'autre part, par le fait que l'on prévoit, selon l'invention, que la branche formant la partie femelle soit à section droite quelque peu en forme de C, à ailes élastiquement déformables, la branche constituant la partie mâle étant à section droite trapézoïdale, à coins arrondis, les branches étant conformées de manière telle que la branche constituant la partie mâle puisse être introduite par effet de déclic dans la branche formant la partie femelle, et par le fait que ledit moyen de sécurité est constitué par un étrier à ailes légèrement élastiques, prévues à l'extrémité libre de la branche conformée en partie femelle, par découpe des ailes du C, suivant deux fentes, dirigées perpendiculairement auxdites ailes.

L'invention sera bien comprise par la description qui suit, faite à titre d'exemple et en référence au dessin annexé, dans lequel :

— la figure 1 est une vue en élévation latérale d'un clamp perfectionné selon l'invention ;

— la figure 2 en est une vue de dessus ;

— la figure 3 est une vue en coupe selon la ligne 3-3 de la figure 2 mais à échelle notablement plus grande ;

— la figure 4 est une vue en coupe selon la ligne 4-4 de la figure 2 et à même échelle que celle de la figure 3 ;

— la figure 5 est une vue à encore plus grande échelle de la zone d'articulation des deux branches du clamp ;

— la figure 6 est une vue en coupe à grande échelle illustrant la condition opératoire de fermeture d'une poche de recueil.

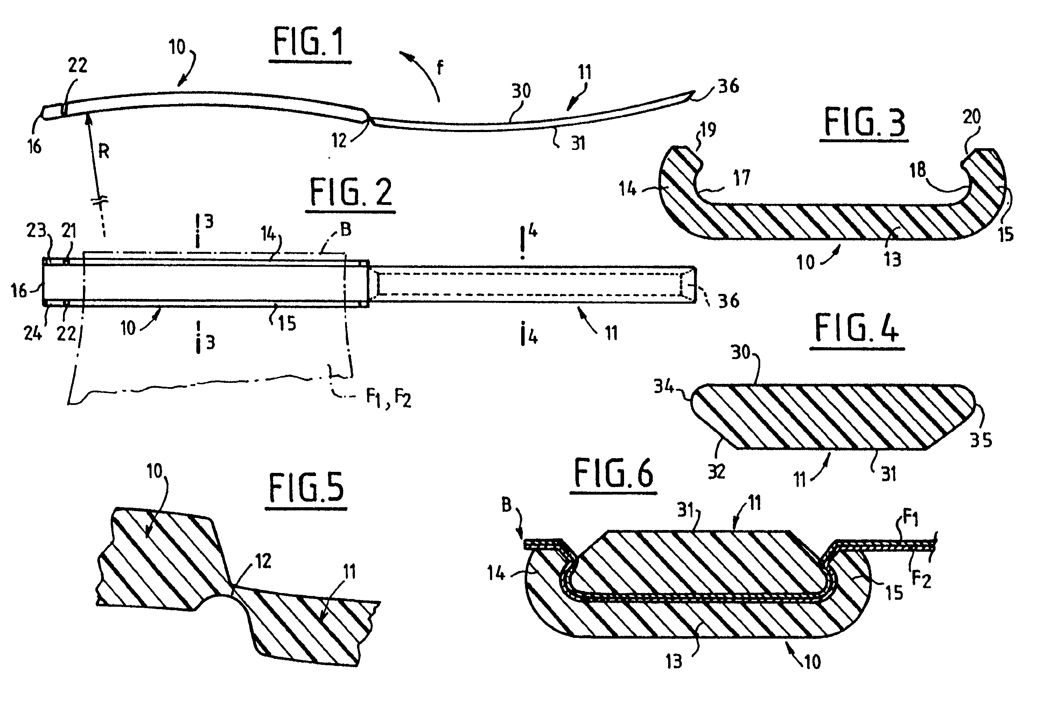

Un clamp selon l'invention, pour la fermeture amovible d'une poche de recueil de déchets corporels, par exemple une poche pouvant être vidangée du type de celles utilisées par des malades ayant subi des interventions comme des iléostomies, est moulé d'une pièce en matière plastique, par exemple un copolymère de polyéthylène haute densité comme celui vendu sous la dénomination "LACQTENE HD" par la Société ATOCHEM (Paris). Comme bien montré sur les figures 1 et 2 le clamp comprend deux branches 10 et 11 reliées entre elles par une charnière-film 12, figure 5, chacune des branches étant moulée suivant un contour plan sensiblement rectangulaire avec, comme bien montré sur la figure 1, un profil à fort rayon de courbure, illustré schématiquement par R sur ladite figure 1. Les branches 10 et 11 sont en outre conformées pour constituer, la première, une pièce femelle et la seconde une pièce mâle à sections droites de formes en partie conjuguées. De façon plus précise, la branche 10 ou partie femelle est à section droite en C, figure 3, avec une âme 13 et des ailes 14 et 15 qui règnent sur la quasi totalité de la longueur de la branche 10, de l'extrémité libre 16 de cette dernière jusqu'à la charnière 12. Les ailes 14 et 15 sont reliées à l'âme 13 par des congés 17 et 18, respectivement, et sont conformées chacune, au-delà desdits congés et vers l'intérieur de la pièce, suivant des pans coupés 19 et 20.

Les ailes 14 et 15 règnent sur la quasi totalité de la longueur de la branche 10, à l'exception de deux fentes 21 et 22 ménagées au voisinage de l'extrémité 16 et dirigées sensiblement perpendiculairement aux bords longitudinaux de grandes dimensions de ladite branche, matérialisés par les ailes 14 et 15. Ces fentes 21 et 22 définissent ainsi, à l'extrémité libre de la partie femelle, — c'est-à-dire distante de la charnière d'articulation 12 —, un étrier en U dont les ailes 23 et 24 sont désolidarisées, du point de vue mécanique, des ailes 14 et 15 de la branche 10.

Avec cette dernière est propre à coopérer la branche 11 dont la section droite, figure 4, est de forme sensiblement trapézoïdale avec une base de grande dimension définissant une face 30 et une base de petite dimension définissant une face 31, lesdites faces étant reliées entre elles par des pans coupés 32 et 33 contigüs à la face 31 et des arrondis 34 et 35, respectivement, contigüs à la face 30 et de même rayon que les congés 17 et 18. A son extrémité libre, c'est-à-dire opposée à la charnière 12, la branche 11 dont la longueur développée est légèrement supérieure à celle de la branche 12 présente un méplat 36 sur sa face 31.

Le choix du matériau constitutif du clamp, de même que les formes et dimensions des branches de celui-ci sont telles que la branche 11 peut être introduite par effet de déclic dans la branche 10 lorsque, par pivotement de la branche 11 autour de l'articulation 12 dans le sens montré par la flèche f sur la figure 1, on amène d'abord ladite branche au contact des ailes 14 et 15 de la branche 10 puis que l'on continue ce mouvement en sollicitant lesdites ailes à l'écartement, par coopération des parties arrondies comme 34 et 35 avec les pans coupés 19 et 20. A la fin du mouvement du pivotement l'extrémité libre de la branche 11 vient se loger, avec effet de déclic, dans l'étrier formé par les ailes 23 et 24 de l'extrémité libre de la branche 10, la partie de la branche 11 correspondant au méplat 36 étant alors en légère saillie au-delà du bord libre de la branche 10.

Lorsque, préalablement à la fermeture du clamp comme décrite immédiatement ci-dessus, on place sur l'aile 10 les deux feuilles ou films $F_1$, $F_2$, figure 6, de la poche de recueil, dans la partie du bord libre B de cette dernière qui définit l'orifice de vidange, l'actionnement décrit amène le pincement à étanchéité des deux dites feuilles ou films entre la pièce femelle 10 et la pièce mâle 11, c'est-à-dire la fermeture étanche de la poche de recueil des déchets corporels.

L'ouverture du clamp est effectuée par un mouvement inverse de celui décrit précédemment, en prenant appui sur la face 30 de la branche 11, dans la zone du méplat 36 dépassant de la branche 10, pour extraire ladite zone des ailes 23 et 24 puis, progressivement, le reste de la branche 11 de la branche 10. A la fin du pivotement d'ouverture les deux films ou feuilles $F_1$, $F_2$ du bord libre de la poche sont libérés de sorte que la poche peut être vidangée sans qu'elle ait à être détachée de l'ouverture d'ostomie de l'utilisateur.

On peut ainsi procéder à une vidange à plat de ladite poche, un tel mode de fonctionnement étant particulièrement avantageux lorsque la poche de recueil est utilisée lors de soins post-opératoires, ou à la suite d'interventions chirurgicales comme des iléostomies.

Le clamp selon l'invention, de faible poids et épaisseur, est particulièrement bien adapté à la fermeture d'une poche de recueil portée sous des vêtements qui exercent par leur configuration sur la poche et le clamp une certaine force de pression. Dans un tel cas, la structure du clamp perfectionné selon l'invention autorise une déformation du clamp, en raison de sa souplesse inhérente, déformation qui peut, par une modification du rayon de courbure R, parfaitement adapter le clamp à la morphologie de la cuisse, ou de l'abdomen du patient utilisant la poche, la modification de ce rayon de courbure provoquant un léger déplacement relatif longitudinal d'une branche par rapport à l'autre, sans qu'il soit porté atteinte à l'étanchéité de la fermeture, laquelle est assurée sur toute la longueur du clamp.

De bons résultats ont été obtenus avec un dispositif selon l'invention dans lequel le rayon de courbure R des deux branches 10, 11 du clamp était d'environ 200 mm, la longueur développée de la branche 10 d'environ 81 mm et celle de la branche 11 d'environ 86 mm. Dans un tel clamp l'épaisseur de la branche 11 était de l'ordre de 2,2 mm et sa largeur d'environ 8 mm, le dispositif de clamp étant utilisé pour la fermeture de poches de recueil dont l'épaisseur du film constitutif, dans la zone des bords libres, était de l'ordre de 60 à 80 μ pour chacun des films, c'est-à-dire une épaisseur à serrer par pincement d'environ 120 à 160 μ.

**Revendications**

1. Clamp pour la fermeture d'une poche de recueil de déchets corporels, moulé d'une pièce en matière plastique et comportant deux branches (10, 11) articulées entre elles autour d'une charnière-film (12), lesdites branches étant conformées pour assurer, en condition opératoire, le serrage entre elles des feuilles ou films constitutifs de la poche sur le bord libre de vidange de celle-ci, avec des moyens d'immobilisation amovible des branches l'une par rapport à l'autre, lesdites branches (10, 11) à contour plan sensiblement rectangulaire étant conformées suivant les parties mâle et femelle à section droite en partie conjuguée d'un accouplement à engagement, d'une part, et ménageant à leur extrémité opposée à celle d'articulation (12) un moyen de sécurité à engagement à pincement élastique, d'autre part, caractérisé en ce que la branche (10) formant la partie femelle est à section droite quelque peu en forme de C, à ailes (14, 15) élastiquement déformables, la branche (11) constituant la partie mâle étant à section droite trapézoïdale à coins arrondis, les branches (10, 11) étant conformées de manière telle que la branche (11) constituant la partie mâle puisse être introduite par effet de déclic dans la branche (10) formant la partie femelle, et en ce que ledit moyen de sécurité est constitué par un étrier (23, 24), à ailes légèrement élastiques, prévu à l'extrémité libre de la branche conformée en partie femelle (10) par découpe des ailes (14, 15) du C suivant deux fentes (21, 22) dirigées perpendiculairement auxdites ailes (14, 15).

**Patentansprüche**

1. Klammer zum Schließen einer Sammeltasche für Abfälle des Körpers, geformt aus einem Stück aus Kunststoffmaterial, zwei Schenkel (10, 11) umfassend, die untereinander um einen Scharnierfilm (12) gelenkig sind, wobei diese Schenkel so geformt sind, daß sie in Arbeitsstellung das Zwischen-sich-Einklemmen der die Tasche bildenden Folien oder Filme gegen den freien Entleerungsrand dieser sicherstellen, mit Einrichtungen zum Unbeweglichmachen der Schenkel bezüglich einander, wobei diese Schenkel (10, 11) mit planer im wesentlichen rechtwinkeliger Kontur entsprechend dem männlichen und dem weiblichen Teil mit geradem zum Teil konjugierten Profil einer Eingriffskupplung einerseits gestaltet sind und an ihrem dem Gelenkende (12) gegenüberliegenden Ende ein Sicherheitsmittel mit elastischem Klemmeingriff andererseits aufweisen, dadurch gekennzeichnet, daß der den weiblichen Teil bildende Schenkel (10) von geradem Profil mit etwas C-Gestalt ist, mit elastisch verformbaren Flügelteilen (14, 15), wobei der den männlichen Teil bildende Schenkel (11) von Trapezprofil mit abgerundeten Ecken ist, wobei die Schenkel (10, 11) derart ausgestaltet sind, daß der den männlichen Teil bildende Schenkel (11) mittels Rasteffekt in den den weiblichen Teil bildenden Schenkel (10) eingeführt werden kann und daß dieses Sicherheitsmittel gebildet wird durch einen Bügel (23, 24) mit leicht elastischen Flügelteilen, der am freien Ende des als weiblicher Teil (10) ausgebildeten Schenkel durch Abschneiden der Flügelteile (14, 15) des C entsprechend zwei Schlitzen (21, 22) vorgesehen ist, die senkrecht zu diesen Flügelteilen (14, 15) gerichtet sind.

**Claims**

1. Clamp for the closure of a bag for collecting body waste, moulded from a piece of plastics material and comprising two arms (10, 11) articulated inter se about a film hinge (12), the said arms being shaped so that in the conditions of use, they ensure that the sheets or films constituting the bag are clamped between them along the free edge over which the bag is drained, comprising means for the detachable immobilisation of the arms with respect to each other, the said arms (10, 11) of substantially rectangular flat contour being shaped according to the male and female parts with a cross-section in the part connected with an engagement coupling on the one hand, and providing, at their end opposite this articulation (12) a securing means of the flexible-grip type and, on the other hand, characterised in that the arm (10) forming the female part is of a substantially C-shaped cross-section having resiliently deformable wings (14, 15), the arm (11) forming the male part being, in cross-section, trapezoidal with rounded corners, the arms (10, 11) being shaped in such a way that it is then possible for the arm (11) forming the male part to be clicked into the arm (10) forming the female part ; and in that the said securing means comprises a stirrup-shaped member (23, 24) having slightly resilient wings, disposed at the free end of the arm which is shaped as a female part (10) by cutting the wing members (14, 15) of the C according to two slits (21, 22) which are directed at right-angles to the said wings (14, 15).

FIG.1

FIG.2

FIG.3

FIG.4

FIG.5

FIG.6

EP 0 278 816 B1